# EUROPEAN PATENT SPECIFICATION

(11) **EP 0 482 787 B1**
(45) Date of publication and mention of the grant of the patent: **27.12.1995**
(21) Application number: 91309235.9
(22) Date of filing: 08.10.1991
(51) Int. Cl.: C07C 51/47, C07C 51/573

(54) **Process for removing iodide compounds from carboxylic acids and/or their anhydrides**
Verfahren zur Abtrennung von Iodidverbindungen aus Carbonsäuren und/oder deren Anhydriden
Procédé d'enlèvement de composés iodures des acides carboxyliques et/ou leurs anhydrides

(30) Priority: 19.10.1990 GB 9022787
(43) Date of publication of application: 29.04.1992
(73) Proprietor: THE BRITISH PETROLEUM COMPANY P.L.C., London EC2M 7BA (GB)
(72) Inventor: Jones Michael David, Sunbury-on-Thames, Middlesex TW16 7LN (GB)
(74) Representative: Barlow, Michael Thomas

(56) References cited:
- EP-A- 0 296 584
- EP-A- 0 361 785
- GB-A- 2 112 394
- US-A- 4 615 806

## Description

The present invention relates to a process for removing iodide compounds, eg alkyl iodides, from carboxylic acids and/or carboxylic acid anhydrides. In particular the present invention is suited to purifying acetic acid and/or acetic anhydride prepared by the rhodium catalysed, methyl iodide promoted carbonylation of methanol and/or methyl acetate.

It is known that a problem associated with acetic acid and/or acetic anhydride produced by carbonylation of methanol and/or methyl acetate in the presence of a rhodium/methyl iodide catalyst system is that even after distillation, the acetic acid and/or acetic anhydride frequently contains small amounts of iodide impurities. Whilst the exact nature of these compounds is not known for certain, they probably comprise a mixture of methyl iodide and other higher alkyl iodides, HI and iodide salts. Such impurities are particularly troublesome since they poison many of the catalysts which are employed in subsequent chemical conversions of the acetic acid and/or acetic anhydride. A case in point are the catalysts used to prepare vinyl acetate from ethylene and acetic acid which are extremely sensitive to iodide impurities.

Several methods of removing iodide compounds from acetic acid and/or acetic anhydride are known. GB 2112394A, for example, teaches the use of anion exchange resins. US 4615806 discloses the removal of iodide impurities from non aqueous organic media such as acetic acid by the use of a silver or mercury containing macroreticular strong acid cation exchanged resin such as Amberlyst 15 (Amberlyst is a Registered Trade Mark).

EP 296584 also teaches using silver exchanged macroreticular resins to purify acetic acid contaminated with iodide impurities.

Such resins are not entirely satisfactory.

It has now been surprisingly found that certain ion exchange resins having functional groups with sulphur donor atoms can be used for removing iodide impurities from carboxylic acids and/or carboxylic acid anhydrides.

Thus according to the present invention there is provided a process for removing iodide compounds from liquid carboxylic acid having 2 to 6 carbon atoms and/or its corresponding carboxylic acid anhydride with an ion exchange resin characterised in that the resin has functional groups which each have at least one sulphur donor atom and which resin has at least some of the sulphur-donor functional groups occupied by silver, palladium and/or mercury, preferably silver.

The ion exchange resin may be of the gel, mesoporous or macroporous type, but is preferably of the mesoporous or macroporous type.

The resin may be any suitable polymer backbone, for example a resin based on a cross-linked polyacrylic or polystyrene matrix.

Suitable functional groups on the ion exchange resin prior to loading with silver, palladium and/or mercury comprise thiol groups (-SH) and substituted thiol groups (-SR) where R is a hydrocarbyl group or substituted hydrocarbyl group.

Suitable thiol functional groups include simple thiol groups -SH; aliphatic thiol groups, for example: 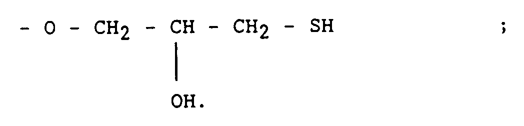
aryl thiol groups, for example: 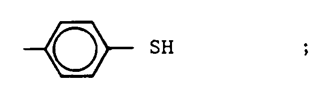
alicyclic thiol groups, for example: 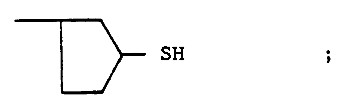
and thiouronium groups, for example:

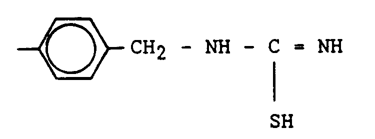

Suitable substituted thiol functional groups include isothiouronium groups, for example: 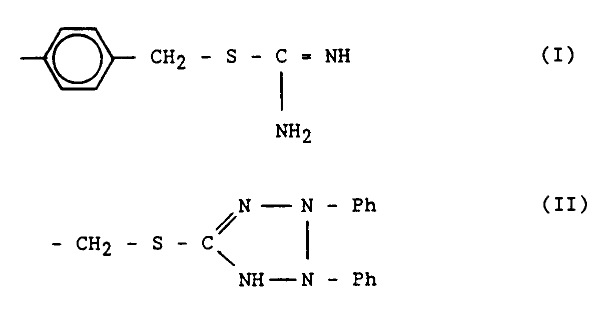
and include thiadiazole groups, for example: 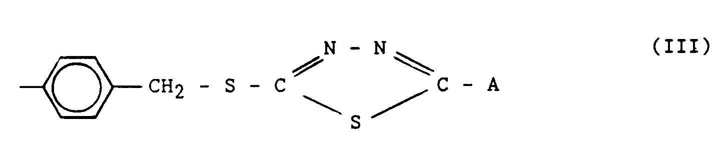
where - A is -NH₂ or -SH.

Preferably, the functional groups in the ion exchange resin prior to loading with silver, palladium and/or mercury comprise thiol groups. These are believed to be only weakly acidic.

It will be understood that other functional groups may be present on the resin. For example if the resin has thiol functional groups, some sulphonic acid groups may be present for example, as a result of oxidation of the thiol groups.

It is believed that silver, palladium and/or mercury-loaded resins with functional groups having sulphur donor atoms may be less succeptible to displacement of the silver, palladium and/or mercury by other metals, for example corrosion metals, than other silver, palladium and/or mercury-loaded resins for example, silver loaded strong acid cation-exchanged resins.

A suitable resin for use in the present invention is Duolite GT73 formally known as Imac TMR and Imac GT-73 (trade marks) available from Rohm and Haas. This is a macroporous ion exchange resin based on a crosslinked polystyrene matrix, having aryl thiol functional groups with a minor amount (generally less than 20%) of sulphonic acid groups.

Another resin suitable for use in the present invention is Lewatit TP214 or Lewatit OC-1014 (Lewatit is a Trade Mark) which is available from Bayer. This resin is based upon a crosslinked polystyrene matrix with thiouronium functional groups.

Yet another resin suitable for use in the present invention is Purolite S920 (Purolite is a Trade Mark) available from Purolite International which has isothiouronium functional groups.

Spheron 1000 available from Lachema is a resin suitable for use in the present invention which has aliphatic thiol functional groups

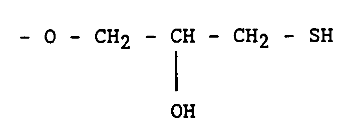

A resin having thiadiazole functional groups suitable for use in the present invention may be prepared by the reaction of 1,3,4-thiadiazole-2,5-dithiol with chloromethylated polystyrene Amberlite XE 305. The functional groups in this resin have the formula:

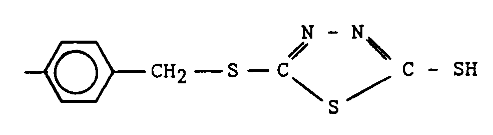

Another suitable resin is Duolite ES 465 which has thiol functional groups.

The amount of silver, palladium and/or mercury present in the resin to be used is suitably such that at least one percent of the sulphur-donor functional groups are occupied by the metal, preferably at least 20% of the groups, more preferably at least 30%.

The metal-loaded resins can be prepared by ion-exchange or impregnation techniques, known in the art. A preferred method for preparing silver-loaded resin is that described in EP 296584A. This method involves slurrying a sample of the resin with silver oxide in water and thereafter treating the slurry with carboxylic acid. A similar method may be used for preparing mercury-loaded resins from mercury (II) acetate. Palladium-loaded resins may be similarly prepared from palladium acetate but without the addition of water other than that used for washing the resin.

The process of the present invention may be carried out as a batch or continuous process.

The process of the present invention is suitably carried out by passing the carboxylic acid or carboxylic acid anhydride contaminated with iodide compounds through a fixed or moving bed of the resin at a predetermined rate. Preferably the resin bed is a fixed bed. The rate used will depend on a number of variables including the amount of iodide compounds in the liquid carboxylic acid or carboxylic acid anhydride, the degree of purity required and the resin employed. Typical flow rates are in the range 0.5 to 30 bed volumes per hour, preferably 5 to 15.

The temperature at which the process is carried out must be high enough to prevent the liquid carboxylic acid or carboxylic acid anhydride from freezing at one extreme or boiling at the other. Typical ranges for atmospheric pressure operation are 20 to 110°C preferably 25 to 80°C. The stability of the resin may also impose an upper limit to the operating temperature.

The process of the present invention is particularily suitable for removing iodide compounds from carboxylic acids having 2 to 6 carbon atoms and their corresponding anhydrides and mixed anhydrides, preferably acetic acid, propionic acid, acetic anhydride and propionic anhydride.

The iodide compounds may be C₁ to C₁₀ alkyl iodide compounds, HI or iodide salts. The process of the present invention is particularly suitable for removing C₁ to C₁₀ alkyl iodide compounds such as methyl iodide and/or hexyl iodide.

The process of the present invention will now be illustrated by the following Examples.

### RESIN PREPARATION

### Example 1 - Preparation of Silver-Loaded Duolite GT73

24.4g wet Duolite GT73 was washed with three 20ml aliquots of water, each washing lasting 20 minutes in a vessel fitted with a PTFE stirrer operating at 60 rpm.

To the washed resin was added silver (I) oxide powder (1.9g) and 20ml water, the slurry being stirred for 30 minutes at room temperature before acetic acid (15ml) was added and the mixture heated to 50°C for 3 hours with the stirrer at 60 rpm.

After this period the resin was filtered, washed with two, 50ml aliquots of acetic acid and dried under an air flow. The silver-loaded resin was analysed as containing 12% by weight silver.

### Example 2 - Preparation of Silver-Loaded Lewatit TP214

Example 1 was repeated using Lewatit TP214 and similar weights of reagents. The silver-loaded resin was analysed as containing 7.2% by weight silver.

### Example 3 - Preparation of Mercury-loaded Duolite GT73

Example 1 was repeated using 25g of Duolite GT73 and 5.2g of mercury (II) acetate.

### Comparative Example A - Preparation of Silver-Exchanged Purolite CT175

Example 1 was repeated using Purolite (Purolite is a Trade Mark) CT175 and similar weights of reagents. This resin is a strong acid cation resin and does not have functional groups with sulphur donor atoms. Therefore this resin is not according to the present invention. The silver-loaded resin was analysed as containing 11.3% by weight silver.

### BATCH TESTING OF SILVER-LOADED RESINS WITH ACETIC ACID

### Example 4 - Batch Testing of Silver-Loaded Duolite GT73

1ml (wet settled volume) of silver-loaded Duolite GT73 prepared in Example 1 was placed in a sealed glass vessel provided with a magnetic stirrer. A 5ml solution of acetic acid containing methyl iodide (about 2 mmol) was added to the vessel together with tetrahydropyran (about 0.3g accurately weighed, internal standard). The solution in the vessel was sampled and analysed for methyl iodide by gas chromotography before being immersed in a water bath heated to 40°C. At regular intervals the vessel was cooled, the solution sampled and analysed for methyl iodide, and the vessel was returned to the water bath. The progress of the uptake of methyl iodide by the silver-loaded resin was monitored by the decrease in methyl iodide concentration and increase in methyl acetate concentration relative to the internal standard.

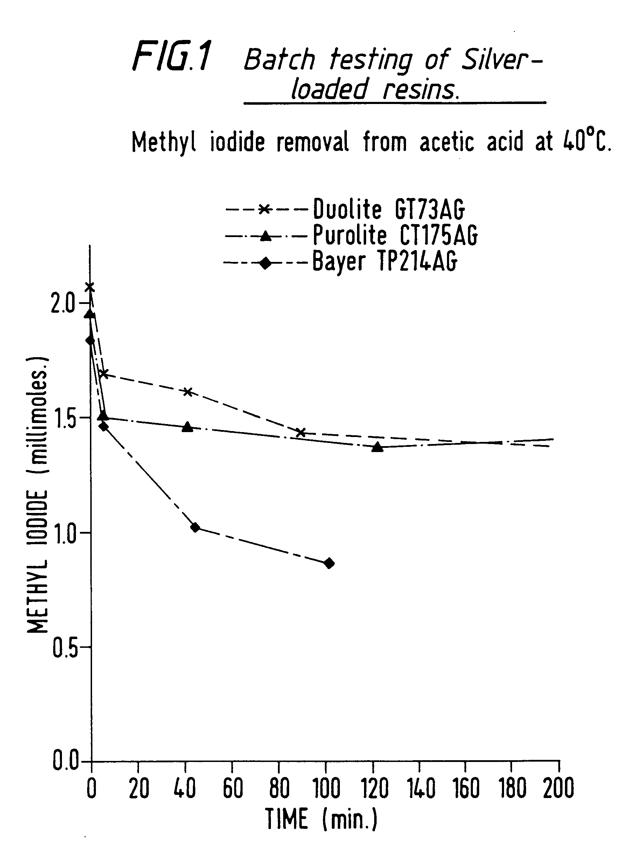

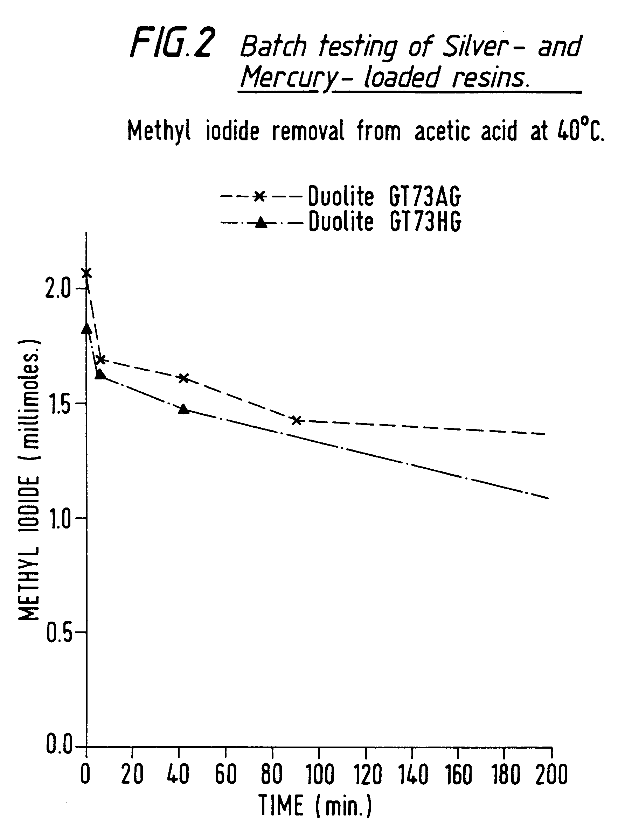

The results are shown in graph form in Figure 1 and Figure 2.

### Example 5 - Batch Testing of Silver-Loaded Lewatit TP214

Example 4 was repeated using the silver-loaded Lewatit TP214 prepared in Example 2. The results are shown in graph form in Figure 1.

### Example 6 - Batch Testing of Mercury-Loaded GT73

Example 4 was repeated using the mercury-loaded Duolite GT73 prepared in Example 3. The results are shown in graph form in Figure 2.

### Comparative Example B - Batch Testing of Silver-Exchanged Purolite CT175

Example 4 was repeated using the silver-exchanged Purolite CT175 prepared in Comparative Example A and the results are also shown in Figure 1. This is not an example according to the present invention because the resin does not have functional groups with sulphur donor atoms.

Referring to Figures 1 and 2, the differences in the rates and amounts of methyl iodide taken up by the resins are affected by how much the resins change in volume during their preparation, since this affects the amount of metal per unit volume of the final resins.

### CONTINUOUS FLOW TESTING OF SILVER-LOADED RESINS WITH ACETIC ACID

### Example 7 - Continuous Flow Testing of Silver-Loaded Duolite GT73

30ml wet settled volume silver-loaded Duolite GT73 prepared in Example 1 was charged as a slurry to a glass column half-filled with acetic acid. The resin bed was backwashed with acetic acid then warmed to 80°C using a circulating water jacket. Acetic acid was passed through the resin bed at 5 bed volumes per hour to warm the bed and for 30 minutes thereafter to stabilise the bed temperature Acetic acid containing about 4000 ppm methyl iodide was then passed through the resin bed at 5 bed volumes per hour and the product was analysed for methyl iodide content by gas chromatography analysis.

Breakthrough was measured at the first indication of methyl iodide in the product acid and at which point the resin was still operating at about 95% iodide impurity removal. This gave a measure of the resin capacity. The results are shown in Table 1. The product acid contained less than 0.05 micrograms per ml silver.

### Comparative Example C - Continuous Flow Testing of Silver-Exchanged Purolite CT175

Example 7 was repeated using silver-exchanged Purolite CT175 prepared in Comparative Example A. The results was shown in Table 1.

### Comparative Example D - Continuous Flow Testing of Silver-Exchanged Amberlyst 15

Example 7 was repeated using silver-exchanged Amberlyst 15 prepared by a method similar to Comparative Example A. Amberlyst 15 is a strong acid cation exchange resin.

The results are shown in Table 1.
Referring to Table 1, comparison of the results from Example 7 and Comparative Examples C and D shows that the resins have comparable capacity for iodide impurities although it is expected that this capacity will be affected by the amount of silver in the resins.

**TABLE 1**

| Resin | Run Temp °C | LHSV/h | I feed ppm | I capacity mol/l resin |
|---|---|---|---|---|
| Duolite GT73 | 80 | 5 | 3769 | 0.45 |
| Purolite CT175 | 80 | 5 | 3690 | 0.55 |
| Amberlyst-15 | 80 | 5 | 3690 | 0.38 |

### BATCH TESTING OF SILVER-LOADED RESINS WITH ACETIC ANHYDRIDE

### Example 8 - Batch Testing of Silver-Loaded Duolite GT73

2.5 ml (dry volume) of silver-loaded Duolite GT73 prepared as in Example 1 (12.3 weight % silver) was stirred with 25 ml of acetic anhydride (prepared by rhodium-catalysed carbonylation of methanol/methyl acetate mixture) at room temperature for 4 hours and then at 80°C for a further one hour. The resin was then filtered off and the product anhydride analysed by Neutron Activation Analysis for total iodide content. The total iodide content of the acetic anhydride before treatment was 524 ± 13 ppb and after treatment was 42.4 ± 2.5 ppb (91.9% removal).

### Example 9 - Batch Testing of Silver-Loaded Lewatit TP214

Example 8 was repeated using silver-loaded Lewatit TP214 prepared as described in Example 2 (7.2 weight % silver). Total iodide content of the acetic anhydride after treatment was 53.4 ± 2.8 (89.8% removal).

### Comparative Example E - Batch Testing of Silver-Loaded Amberlite IR120

Silver-loaded Amberlite IR120 (typically containing 10.5% by weight silver) was prepared as in Example 1 but using Amberlite IR120 resin which is a strong acid cation gel resin Example 8 was repeated using the silver-loaded Amberlite IR120 resin. Total iodide content of the acetic anhydride after treatment was 142.3 ± 4.7 ppb (72.8% removal) and the anhydride product was discoloured.

Examples 8 and 9 and Comparative Example E were performed with acetic anhydride which contained oxidisable impurities. The results show that the presence of such impurities causes a discolouration of the anhydride product when treated with silver-exchanged strong acid cation resins but not with silver loaded resins according to the present invention.

Further experiments (Examples 10-11 and F) were performed with acetic anhydride which had been treated to reduce the oxidisable impurities.

### Example 10 - Batch Testing of Silver-Loaded Duolite GT73

Example 8 was repeated using 12 ml of resin and 120 ml of acetic anhydride.

The iodide content of the anhydride before treatment was 431 ± 10 ppb and after treatment was 7.8 ± 1.3 ppb (98.2% removal). After treatment however, the acetic anhydride was cloudy.

### Example 11 - Batch Testing of Silver-Loaded Lewatit TP214

Example 10 was repeated using Lewatit TP214. The total iodide content of the acetic anhydride after treatment was 6.7 ± 1.3 ppb (98.4% removal). The acetic anhydride product was clear and not cloudy after treatment.

### Comparative Example F - Batch Testing of Silver-Loaded Amberlite IR120

Comparative Example E was repeated using the quantities of reagents as in Examples 10 and 11.

The acetic anhydride after treatement was clear and had a total iodide content of 65.0 ± 2.9 ppb (84.9% removal).

## Claims

1. A process for removing iodide compounds from a liquid carboxylic acid having 2 to 6 carbon atoms and/or its corresponding carboxylic acid anhydride characterised in that the process comprises contacting the liquid with an ion exchange resin having functional groups which each have at least one sulphur-donor atom and which resin has at least 1% of its sulphur-donor functional groups occupied by silver, palladium and/or mercury.

2. A process as claimed in claim 1 characterised in that the carboxylic acid is acetic acid and the corresponding carboxylic acid anhydride is acetic anhydride.

3. A process as claimed in claim 1 or claim 2 characterised in that the resin has thiol or substituted thiol functional groups.

4. A process as claimed in claim 3 characterised in that the thiol groups comprise -SH groups, aliphatic thiol groups, aryl thiol groups, alicyclic thiol groups and/or thiouronium groups.

5. A process as claimed in claim 4 characterised in that the thiol groups have the formula: -SH;

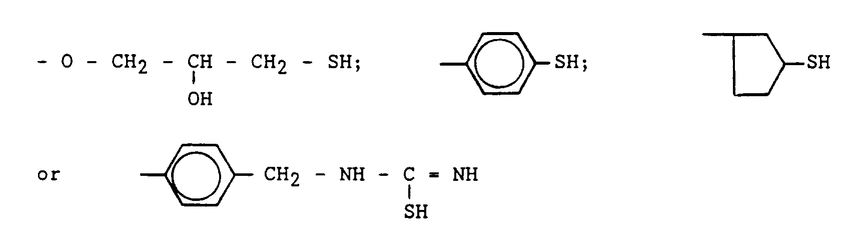

6. A process as claimed in claim 3 characterised in that the substituted thiol groups comprise isothiouronium groups or thiadiazole groups.

7. A process as claimed in claim 6 characterised in that the substituted thiol groups have the formula (I), (II) or (III): 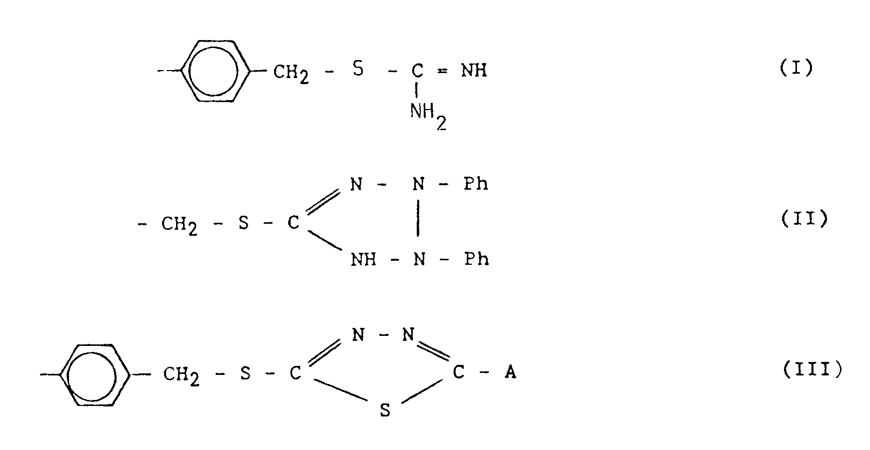 where - A is - NH₂ or - SH

8. A process as claimed in any one of the preceeding claims characterized in that at least 1% of the sulphur-donor functional groups are occupied by silver.

9. A process as claimed in any one of the preceeding claims characterised in that the resin comprises a macroporous ion exchange resin based upon a crosslinked polystyrene matrix.

10. A process according to any one of the preceeding claims characterized in that the iodide compounds comprise C₁ to C₁₀ alkyl iodides.

11. A process according to claim 1 for removing C₁ to C₁₀ alkyl iodides from liquid acetic acid and/or acetic anhydride characterized in that the process comprises contacting the liquid with a macroporous ion exchange resin based on a crosslinked polystyrene matrix having aryl thiol functional groups, at least 1% of which are occupied by silver.

## Patentansprüche

1. Verfahren zur Entfernung von Iodidverbindungen aus einer flüssigen Carbonsäure mit 2 bis 6 Kohlenstoffatomen und/oder ihres entsprechenden Carbonsäureanhydrids, **dadurch gekennzeichnet**, daß das Verfahren das In-Kontaktbringen der Flüssigkeit mit einem Ionenaustauscher-Harz mit funktionellen Gruppen umfaßt, von denen jede zumindest ein Schwefeldonor-Atom hat, und welches Harz zumindest 1 % seiner Schwefeldonor-funktionellen Gruppen durch Silber, Palladium und/oder Quecksilber besetzt hat.

2. Verfahren nach Anspruch 1, **dadurch gekennzeichnet,** daß die Carbonsäure Essigsäure und das entsprechende Carbonsäureanhydrid Essigsäureanhydrid ist.

3. Verfahren nach einem der Ansprüche 1 oder 2, **dadurch gekennzeichnet,** daß das Harz Thiol- oder substituierte Thiol-funktionelle Gruppen besitzt.

4. Verfahren nach Anspruch 3, **dadurch gekennzeichnet,** daß die Thiolgruppen -SH-Gruppen, aliphatische Thiolgruppen, Arylthiolgruppen, alicyclische Thiolgruppen und/oder Thiouroniumgruppen umfassen.

5. Verfahren nach Anspruch 4, **dadurch gekennzeicnet,** daß die Thiolgruppen die nachstehenden Formeln besitzen: -SH;

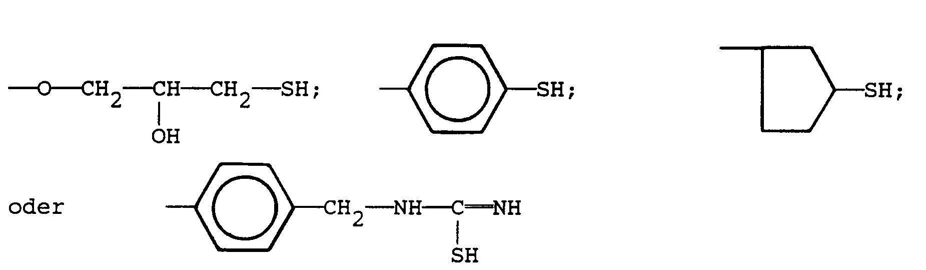

6. Verfahren nach Anspruch 3, **dadurch gekennzeichnet,** daß die substituierten Thiolgruppen Isothiouroniumgruppen oder Thiadiazolgruppen enthalten.

7. Verfahren nach Anspruch 6, **dadurch gekennzeichnet,** daß die substituierten Thiolgruppen die Formeln (I), (II) oder (III) aufweisen: 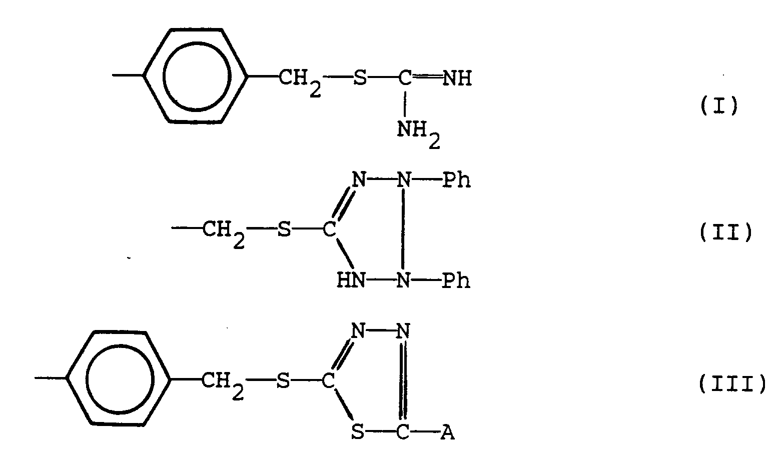 worin A die Bedeutung -NH₂ oder -SH aufweist.

8. Verfahren nach einem der vorhergehenden Ansprüche, **dadurch gekennzeichnet,** daß zumindest 1 % der Schwefeldonor-funktionellen Gruppen durch Silber eingenommen sind.

9. Verfahren nach einem der vorhergehenden Ansprüche, **dadurch gekennzeichnet**, daß das Harz ein makroporöses Ionenaustauscher-Harz, auf Basis einer vernetzten Polystyrol-Matrix, umfaßt.

10. Verfahren nach einem der vorhergehenden Ansprüche, **dadurch gekennzeichnet**, daß die Iodidverbindungen C₁₋₁₀-Alkyliodide enthalten.

11. Verfahren gemäß Anspruch 1 zur Entfernung von C₁₋₁₀-Alkyliodiden aus flüssiger Essigsäure und/oder aus Essigsäureanhydrid, **dadurch gekennzeichnet**, daß das Verfahren das In-Kontakt-bringen der Flüssigkeit mit einem makroporösen Ionenaustauscher-Harz, basierend auf einer vernetzten Polystyrol-Matrix mit Arylthiol-funktionellen Gruppen, von denen zumindest 1 % durch Silber eingenommen sind, umfaßt.

## Revendications

1. Procédé pour éliminer des iodures présents dans un acide carboxylique liquide ayant 2 à 6 atomes de carbone et/ou son anhydride d'acide carboxylique correspondant, caractérisé en ce qu'il comprend la mise en contact du liquide avec une résine échangeuse d'ions ayant des groupes fonctionnels qui portent chacun au moins un atome de soufre pouvant être cédé, résine qui possède au moins 1 % de ses groupes fonctionnels donneurs de soufre occupé par de l'argent, du palladium et/ou du mercure.

2. Procédé suivant la revendication 1, caractérisé en ce que l'acide carboxylique est l'acide acétique et l'anhydride d'acide carboxylique correspondant est l'anhydride acétique.

3. Procédé suivant la revendication 1 ou la revendication 2, caractérisé en ce que la résine porte des groupes fonctionnels thiol ou thiol substitués.

4. Procédé suivant la revendication 3, caractérisé en ce que les groupes thiol comprennent des groupes -SH, des groupes thiol aliphatiques, des groupes thiol aryliques, des groupes thiol alicyliques et/ou des groupes thiouronium.

5. Procédé suivant la revendication 4, caractérisé en ce que les groupes thiol répondent à la formule :

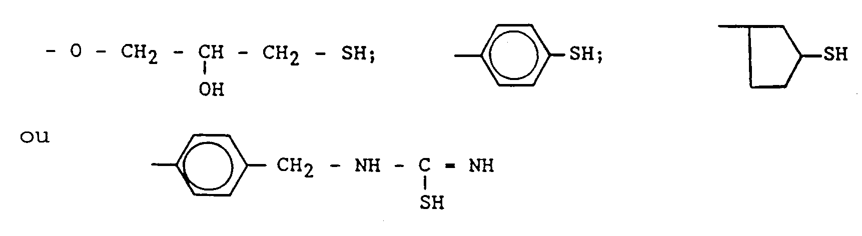

6. Procédé suivant la revendication 3, caractérisé en ce que les groupes thiol substitués comprennent des groupes isothiouronium ou des groupes thiadiazole.

7. Procédé suivant la revendication 6, caractérisé en ce que les groupes thiol substitués répondent à la formule (I), (II) ou (III) : 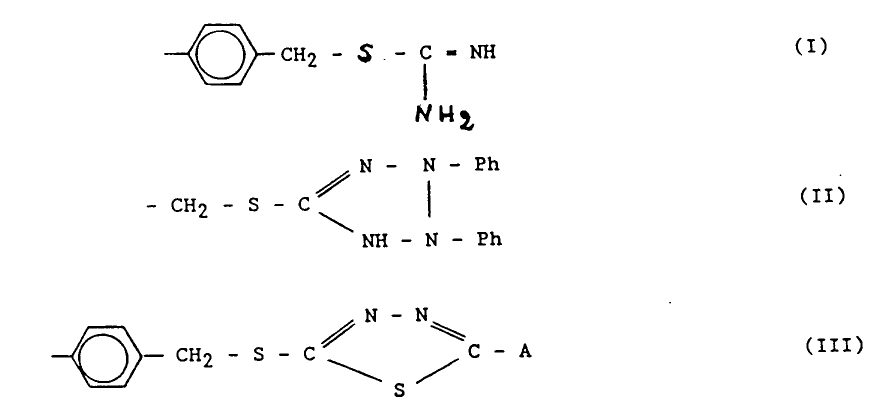 dans laquelle A représente un groupe -NH₂ ou -SH.

8. Procédé suivant l'une quelconque des revendications précédentes, caractérisé en ce qu'une quantité d'au moins 1 % des groupes fonctionnels donneurs de soufre est occupée par de l'argent.

9. Procédé suivant l'une quelconque des revendications précédentes, caractérisé en ce que la résine consiste en une résine macroporeuse échangeuse d'ions à base d'une matrice de polystyrène réticulée.

10. Procédé suivant l'une quelconque des revendications précédentes, caractérisé en ce que les iodures consistent en iodures d'alkyle en C₁ à C₁₀.

11. Procédé suivant la revendication 1 pour éliminer des iodures d'alkyle en C₁ à C₁₀ présents dans de l'acide acétique liquide et/ou de l'anhydride acétique liquide, caractérisé en ce qu'il comprend la mise en contact du liquide avec une résine macroporeuse échangeuse d'ions à base d'une matrice de polystyrène réticulée portant des groupes fonctionnels thiol aryliques, dont au moins 1 % sont occupés par de l'argent.
